# EUROPEAN PATENT APPLICATION

(11) **EP 4 074 352 A1**
(43) Date of publication of application: **19.10.2022**
(21) Application number: 21167907.1
(22) Date of filing: 12.04.2021
(51) Int. Cl.: A61M 1/36, A61B 17/11, A61B 17/00, A61B 90/00

(54) **DEVICES FOR FORMING PERCUTANEOUS VASCULAR ANASTOMOSIS, IN PARTICULAR PERCUTANEOUS ARTERIOVENOUS FISTULA FOR HEMODIALYSIS**

(71) Applicant: Mallios Consulting, 75013 Paris (FR)
(72) Inventor: MALLIOS, Alexandros, 75013 Paris (FR)
(74) Representative: Demulsant, Xavier

(57) **Abstract**

Device for creating a fistula between two vessels, the device comprising two catheters (1, 10), each catheter having a proximal end and a distal end, the two catheters comprising corresponding surface contact areas in their distal ends, the surface contact area of a first catheter (1) extending in the tip (3) of the first catheter (1), the corresponding surface contact area of the second catheter (10) comprising a bearer (12) for the tip (3) of the first catheter (1), characterized in that the bearer (12) extends laterally in the distal end (11) of the second catheter(10), the two catheters (1, 10) comprising indexing means enabling precise relative positioning of the two catheters (1, 10) when the surface contact area of the first catheter (1) is against the surface contact area of the second catheter (10).

## Description

### FIELD OF THE INVENTION

The invention generally relates to internal medical procedures performed percutaneously.

The invention relates to devices and methods for forming percutaneous vascular anastomosis, in particular percutaneous arteriovenous fistula (pAVF) for hemodialysis.

### BACKGROUND OF THE INVENTION

Anastomosis is a surgical connection made between two hollow organs such as blood vessels. The traditional surgical procedure involves hand-suturing the two vessels together such that the end of one vessel is fixedly attached to an opening of the second vessel. This approach is time consuming and requires a surgical incision to provide access and control to the vessels involved.

The arteriovenous fistula (AVF) is the preferred access for hemodialysis. AVFs are less prone to infection and are more durable than both tunneled catheters and arteriovenous grafts.

The predominant method of creating an AVF with a sutured anastomosis has remained unchanged since the original description by *Brescia et al.* in 1966. A surgical procedure generates openings in the vein and the artery that are eventually connected with an anastomosis creating thus the fistula. In some instances, fistula is formed between the brachial artery and the antecubital vein. Fistula creation can be accomplished by creating an incision in the limb, dissecting the vessels, creating a small opening in both vessels and the suturing or clipping the openings together. Eventually, as the vein and artery heal, the fistula matures, which involves enlargement of the vessel diameter and enhancement of their wall.

Surgical fistula creation has several pitfalls. The radial artery is somewhat small at the wrist, which makes anastomosis technically demanding, especially in smaller patients. A relatively large vein is needed in the immediate vicinity of the radial artery, and such vein is not always present, or may be of bad quality because of previous intravenous perfusions. If a vein more than a centimeter away is mobilized and brought over to the artery, venous kinking that compromises the internal lumen and blood flow can occur.

Even with a precise technique, there may be vessel irritation, spasms or intimal hyperplasia, which can lead to early closure or delayed maturation. Percutaneous procedures present numerous advantages over open surgery, e.g. decreased risk of infection, decreased scarring, decreased patient trauma, reduced amounts and duration of anesthesia, reduced occurrence of complications, during or after the procedure, faster recovering times, reduction in the need for post-procedural pain medications.

Device and methods for percutaneous arteriovenous fistula creation for hemodialysis access can be found in documents WO2012068273, WO2014/059351, WO2014153229, WO2015138998, WO2017124062 in the name of TVA Medical, or in documents WO2018/213626, US10070866, EP2582314, EP2812063 in the name of Avenu Medical, or in documents WO2014/078601, WO2014/022585 in the name of Caymus Medical. WO2021/022090 (Nxt Biomedical) disclose two catheters with magnets mounted thereon and used to provide points of shunt contact or placement, the magnetic attraction pulling tissues and catheters together in a fixed reliable geometric manner. The magnets of the first catheter are arranged such that their polarities are configured to mate with the magnetic features of the second catheter. In use, each of the first and second catheters are either rotated up to 90 degrees toward each other such that their distal ends face each other in an axial alignment. This configuration aligns the two central lumens of the catheters and magnetic features, allowing a tool, such as a needle, RF wire, or sharpened guidewire to be advanced through the first catheter, second catheter or both, to puncture the tissue walls trapped between the magnetic features.

WO97/33522 (Beth), published in 1997, disclose a system comprising two discrete catheters for generating an arteriovenous fistula for hemodialysis access. Each catheter has a pair of rare earth magnets. The attraction between these magnets cause intravascular adjustment in position for the venous catheter and the arterial catheter lying within their individual but immediately adjacent blood vessels. A sliding electrode is actuated to perforate both the arterial and the venous vascular wall.

Percutaneous arteriovenous fistula creation for hemodialysis access has been proposed more than twenty years ago.

However, according to Khawaja et al. (Preoperative assessment for percutaneous and open surgical arteriovenous fistula creation in patients for hemodialysis, Clinical Kidney Journal, 408-417, 2021*),* percutaneous created endovascular AVF's for dialysis access have appeared only in 2014. According to Jones et al. (A review of the current status of percutaneous endovascular arteriovenous fistula creation for hemodialysis access, Cardiovasc Intervent Radiol, 42:1-9, 2019*),* two percutaneous endoAVF system were used in 2018, predominantly in clinical trials. The first is the original Flex device, afterwards called the everlinQ^{®} endoAVF system (TVA Medical), now named WavelinQ^{®} (Becton Dickinson). The second is the Ellipsys^{®} device (Avenu Medical, formerly named Caymus Medical, now part of Medtronic).

In a recent review published in 2021, *Chen et al.* have presented both the Ellipsys and the WavelinQ systems (Endovascular creation of arteriovenous fistulas, 341-350, in Dialysis access management, ISBN 978-3-030-52993-2). Since 2015, these two devices have shown technical success in creation of a dialysis fistula percutaneously with functional patency. Initial studies evaluating the EverlinQ/WavelinQ system were single-arm prospective, noncomparative, non-randomized trials evaluating safety and efficacy.

Both technologies are currently being further evaluated in post commercial registries or monocentric surveys in Europe.

Both the WavelinQ and the Ellipsys system create a side-to-side AVF. This configuration is believed to be associated with less development of intimal hyperplasia. This side-to-side configuration is also believed to be associated with more favorable flow characteristics compared with an end-to-side configuration, notably, less turbulent flow and post-anastomotic stenosis

WavelinQ enables the creation of an AVF between either the proximal radial or ulnar arteries and an adjacent deep vein that drains the upper arm superficial veins. Ellipsys connects the proximal radial artery with the adjacent terminal part of the deep communicating vein.

Using both the WavelinQ and the Ellipsys system, vessels are not clamped, not mobilized and not dissected and are not anastomosed by sutures. Comparable with traditional surgical approaches, a preprocedural ultrasound assessment to evaluate the vascular status is mandatory. During this examination, derivations, such as differences in vessel diameter, high brachial bifurcations or nonexistent perforating veins can be identified. The everlinQ system (TVA Medical) was indicated for the creation of an AVF using the ulnar artery and ulnar vein in patients with minimum artery and vein diameters of 2.0 mm and less than 2.0 mm separation between the artery and vein at the fistula creation site who have chronic kidney disease and need hemodialysis.

The first generation everlinQ system had two 6Fr catheters, which were inserted via the upper arm with the vascular system.

In 2017, a CE-approved 4Fr device has replaced the 6Fr system in Europe. The 4Fr system enables a wrist access either from the radial or the ulnar side if the diameters of the vessel are sufficient, otherwise access vie the brachial vessels is performed.

The WavelinQ ^{®} system (Becton Dickinson) consists of a pair of over-the-wire catheters with magnets arranged to pull them into alignment and opposition.

The venous catheter contains a spring-loaded radiofrequency (RF) electrode for delivery of radiofrequency energy.

The arterial catheter contains a ceramic backstop intended to align with the venous RF electrode.

The catheters have radio opaque elements, forming rotational indicators. Following duplex sonography guided puncture 0.014" guidewires are inserted in both vessels up to the planned site of the anastomosis.

The two catheters are positioned via these wires and fluoroscopic imaging is used to check the correct positioning. Fluoroscopic imaging is preferably set perpendicular to the plane of the target vein and artery to maximize accuracy of the rotational indicators.

The arterial catheter is inserted into the ulnar artery via the brachial artery, and the venous catheter is inserted into the ulnar vein via the brachial vein. The catheters are introduced from the upper arm or wrist in a parallel or antiparallel fashion and are guided to the creation site with fluoroscopic imaging.

The catheters are aligned and rotated before reaching the fistula creation site, so that the electrode and ceramic backstop are facing one another. When the catheters achieve the proper position, the magnets contained in each catheter attract each other, the magnets holding the artery and vein together.

The RF electrode is released from the venous catheter and energized for 0.7 second, vaporizing the tissue and vessel walls between the artery and the vein, creating a side-to-side ulnar vein fistula in the arm. A preset energy of 60W is applied.

The energy creates a small rectangular hole (approximately 5 mm x 1 mm) in the adjoining vessels, creating a nonsurgical, or endovascular AVF. The result is documented by angiography.

Blood then flows from the artery into the veins of the deep venous system and via perforators to the superficial venous system, which include the antecubital vein, basilic vein, and cephalic vein.

The endo AVF procedure using WavelinQ is carried out under conscious sedation and local anesthesia and does not require an overnight stay in hospital. Patients receive intravenous heparin.

Prior to the procedure, patients should undergo Doppler vascular mapping to identify adequate vascular diameters for fistula creation. Imaging guidance is made by fluoroscopy.

Patients must have vessels that are more than 2 mm in diameter, patency of the upper arm veins, and no significant central venous stenosis.

The first clinical results of this procedure have been compiled in the Flex study, a prospective clinical evaluation of total vascular access. From August 2012 to September 2013, the system was used to attempt AVF creation between the proximal ulnar artery and a closely associated ulnar vein in 33 patients (Rajan et al, Percutaneous creation of an arteriovenous fistula for hemodialysis access, JVIR, 2015 Apr; 26(4):484-90, doi: 10.1016/j.jvir.2014.12.018).

In the following Neat study, 60 patients were treated between January 2014 to August 2015, with the 6Fr system, the last participant completing the twelve months study follow-up in August 2016 (Lok et al, Endovascular proximal forearm arteriovenous fistula for hemodialysis access: results of the prospective multicenter novel endovascular access trial Neat, Am J Kidney Dis, 2017 Oct; 70(4):486-497, doi: 10.1053/j.ajkd.2017.03.026).

Results obtained for 8 patients, treated between July 2015 and February 2016 are presented by Radosa et al (Endovascular creation of an arteriovenous fistula for hemodialysis access: first results, Cardiovasc Intervent Radiol, 2017, 40:1545-1551)*.*

In a following Neat study, the everlinQ procedure was performed on 32 patients, from May to November 2016, using the 4Fr system (Berland et al, Endovascular creation of arteriovenous fistulae for hemodialysis access with a 4Fr device: clinical experience from the Ease study, Ann Vasc Surg, 2019 Oct; 60:182-192, doi: 10.1016/j.avsg.2019.02.023*).*

Ellipsys ^{®} (Medtronic) is a device which was developed to create a percutaneous AVF for hemodialysis access using pressure and thermal assistance.

This thermal resistance anastomosis device (TRAD) creates a side-to-side anastomosis between the proximal radial artery and an adjacent deep communicating vein in the proximal forearm.

Thermal resistance anastomosis device (TRAD) for the percutaneous creation of AVF for hemodialysis was presented by Hull et al in 2016 (J Vasc Interv Radiol 2016, doi.org/10.1016/j.jvir.2016.10.033). TRAD was a single catheter venous access system to create fistulae under ultrasound guidance. This device uses applied pressure and thermal resistance to fuse artery and vein adventitia together and then cut an elliptical anastomosis between the proximal radial artery and perforating vein. More precisely, the TRAD device consists of three main components: an access needle, an overthe-wire tissue fusion and cutting catheter and a power controller. The TRAD catheter has a 6-F proximal diameter with opposing active surfaces between the base and the coned 5-F distal tip. The power controller delivers direct current to the catheter heating element that is controlled with feedback from the temperature sensors and a gap sensor detecting the temperature and the opening distance of the catheter. A thumb tab in the handle of the device controls the catheter opening and closing and the fusion pressure.

The Ellipsys procedure is typically performed in an outpatient setting, under loco regional anesthesia nerve block or local anesthesia with or without moderate sedation. The entire procedure is performed using ultrasound guidance, without need for radiation and contrast media.

The procedure starts with a puncture of a superficial vein, either cephalic or basilica vein, on the upper arm to navigate from there through the perforator to the deep venous systems. A standard micropuncture needle and wire are used to obtain retrograde access into the median cubital or brachial vein. The access needle is then advanced intravenously under ultrasound guidance to a position where the proximal radial artery is adjacent.

The needle is then used to puncture the proximal radial artery and the wire is advanced distally into the radial artery.

A 6Fr glide sheath slender sheath (Terumo Medical Corp, New Jersey) is then advanced into the artery. Through this, the TRAD catheter is advanced into the sheath in an open position with the tip of the device in the proximal radial artery and the base in the accessed vein.

The catheter has two distinct plates, of which one is placed in the vein and the other in the artery.

The sheath is then retracted and gentle traction is applied to the Ellipsys catheter until the tip of the device engages the wall of the proximal radial artery, providing tactile resistance to further traction.

The device is then closed and activated to fuse and cut the anastomosis. Electrical impulses of up to 15s are created. The Ellipsys device create an elliptical anastomosis that is roughly 4mm x 2 mm.

Once the anastomosis is created, the device can be removed via the sheath. An immediate percutaneous transluminal angioplasty (PTA) of the anastomosis and the perforating vein is recommended. Such a procedure is not currently used for the WavelinQ system.

In 2017, *Hull et al.* published a prospective sing arm evaluation of the Ellipsys system. Between January 2014 and March 2015, 26 patients were treated. (Thermal resistance anastomosis device for the percutaneous creation of arteriovenous fistulae for hemodialysis, JVIR, 2017 Mar; 28(3):380-387, doi: 10.1016/j.jvir.2016.10.033*).*

In 2018, *Hull et al.* published a prospective single arm trial involving five sites and 103 patients, comparing the Ellipsys device 90 days performance against meta-analysis of surgical results obtained from the literature. Initial venous access was retrograde through the cubital vein or brachial vein. Secondary maturation procedures were performed to create functional fistulas and included balloon dilatation, brachial vein embolization, basilica vein ligation or embolization (The pivotal multicenter trial of ultrasound guided percutaneous arteriovenous fistula creation for hemodialysis access, JVIR, 2018 Feb; 29(2):149-158.e5, doi: 10.1016/j.jvir.2017.10.015).

*Mallios et al.* performed a retrospective review of date from a single center's experience with the Ellipsys system. Creation of the percutaneous AVF was performed in 34 patients (Early results of percutaneous arteriovenous fistula creation with the Ellipsys vascular access system, JVS, 2018 Oct; 68(4):1150-1156, doi: 10.1016/j.jvs.2018.01.036)*.*

*Beathard et al.* described a two year follow up after use of the Ellipsys device at five vascular access programs in the United States with a total of 105 patients. In those patients, percutaneous AVF creation was performed with immediate balloon dilatation of the anastomosis as described by Mallios et al. (Two-year cumulative patency of endovascular arteriovenous fistula, JVS, 2020 May; 21 (3):350-356, doi: 10.1177/1129729819877780)*.*

*Mallios et al.* have recently presented midterms results obtained on 234 patients who had a pAVF created using Ellipsys vascular access system *(*JVS 2020 Dec; 72(6):2097-2106, doi: 10.1016/j.jvs.2020.02.048*).*

Some case reports have been published recently describing the use of Ellipsys system in adolescents (Koo et al. Radiology Case Reports, March 2021, doi.org/10.1016/j.radcr.2020.12.026).

Available endoAVF systems have some disadvantages.

One disadvantage is that correct placement of the system within the vessels and correct puncture of the vessels can largely depend on the experience and skills of the surgeon.

Using the WavelinQ system, the catheters have to be aligned and rotated before reaching the fistula creation site, so that the electrode and ceramic backstop are facing one another. Rotational indicators in the eclipsed position indicate misalignment, whereas the open position indicates alignment. Alignment has to be further confirmed by the communication of the venous electrode with the artery backstop during electrode reciprocation. The need of aligning the catheters for a certain length that is equal or superior to 4-5cm is forcing the connection to be further away from the deep communicating vein. As a result, the flow can be restricted from the in between small radial or ulnar vein or be distributed to other branches that are before the deep communicating vein hence having less flow going to the superficial venous system which is necessary for the fistula maturation and use. Furthermore, tortuosity and branching of the deep venous system may make the alignment of the catheters cumbersome and time consuming or in some occasions not at all possible.

Using the Ellipsys system, the access needle is advanced intravenously under ultrasound guidance to a position where the proximal radial artery is adjacent. The needle is then used to puncture the proximal radial artery. Puncture is made in a direction based on information given by ultrasound guidance. This is a delicate and difficult process that requires the operator under ultrasound to navigate inside the center of a 2-3mm vein, come out of it and then come in a 2mm artery, staying always at the center and avoiding hematoma or spasm that will make visualization under ultrasound difficult. Given that these skills of ultrasound guided puncture are not widely diffused amongst physicians and especially surgeons, many hesitate to perform the technique. Furthermore, during the balloon dilatation of the anastomosis, the balloon comes down from the deep communicating vein into the radial artery and towards the distal radial artery. When the balloon is inflated it will squeeze and compress the proximal radial artery and this can cause a temporary or permanent reduction of antegrade flow in the radial artery that is required for fistula function and maturation. To fix this problem, a second wire needs to be brought from the distal radial artery and with a separate balloon do an angioplasty of the pre-anastomotic radial artery.

Moreover, using the WavelinQ system, the arm to be treated using RF has to be fixed to prevent movement due to myoclonus trigged by radiofrequency impulse-induced neuro stimulation during energy delivery, such movement being more frequent and more severe in creation of an ulnar fistula due to the proximity of the medial nerve. When using EverlinQ/WavelinQ system, a pronounced intraoperative vessel spasm is often seen after high frequency application, possibly trigged by thermal effects, small hematomas or electrical stimulation.

Although many steps of the EverlinQ/WavelinQ procedure can be carried out by ultrasound guidance, angiographic imaging is still required.

Mean procedure time is long for WavelinQ. According to *Shahverdyan,* wavelinQ procedure required 73 minutes mean, with a range between 2 and 150 minutes (Single center experience of endovascular AV fistula creation with both wavelinQ and ellipsys systems, European Journal of Vascular & Endovascular Surgery; 2019 Supplement 3, Vol. 58, pe690-e690, 1p, doi: 10.1016/j.ejvs.2019.09.209)*.*

### SUMMARY OF THE INVENTION

The present invention is directed to addressing the defects of both prior art devices and create a single device that takes care of one or more of the problems set forth above. The following presents a simplified summary of the invention in order to provide a basic understanding of some aspects of the invention. This summary is not an exhaustive overview of the invention. It is not intended to identify key of critical elements of the invention or to delineate the scope of the invention. Its sole purpose is to present some concepts in a simplified form as a prelude to the more detailed description that is discussed later.

While the invention is susceptible to various modification and alternative forms, specific embodiments thereof have been shown by way of example in the drawings. It should be understood, however, that the description herein of specific embodiments is not intended to limit the invention to the particular forms disclosed.

It may of course be appreciated that in the development of any such actual embodiments, implementation-specific decisions should be made to achieve the developer's specific goal, such as compliance with system-related and business-related constraints. It will be appreciated that such a development effort might be time consuming but may nevertheless be a routine understanding for those or ordinary skill in the art having the benefit of this disclosure.

The invention relates to devices and methods for forming percutaneous vascular anastomosis, that can be an arteriovenous fistula (pAVF, endoAVF) for hemodialysis or other type of vascular anastomosis, avoiding the drawbacks and side effects of available systems and methods, and being cost effective, clinically and practically.

In one aspect, the present disclosure relates to a device for creating a fistula between two vessels, the device comprising two catheters, each catheter having a proximal end and a distal end, the two catheters comprising corresponding surface contact areas in their distal ends, the surface contact area of a first catheter extending in the tip of the first catheter, the corresponding surface contact area of the second catheter comprising a bearer for the tip of the first catheter, the bearer extending laterally in the distal end of the second catheter, the two catheters comprising indexing means enabling precise relative positioning of the two catheters when the surface contact area of the first catheter is against the surface contact area of the second catheter. The first catheter is relatively rigid which allows control and navigation of the tip in order for it to be positioned at the desired position. The tip can be equipped with magnet to assist connection with the second catheter or a thermal element to allow for tissue fusion technology to be applied.

Advantageously, the first catheter has an internal lumen having an aperture in said tip for the passage of a tool or a wire that will assist advancement of the catheter and will also create the connection, the second catheter having a lateral guiding surface facing said aperture when the surface contact area of the first catheter is against the surface contact area of the second catheter. The alignment of the catheters will allow the guide wire to go from the lumen of the first catheter to the guiding convex surface of the second catheter and create the connection with the wire advancing in a desired angled and not straight direction.

In some embodiments, the surface contact area of the first catheter is a male surface contact, the tip of the first catheter forming a convex surface area. The second catheter has a female surface contact. These can be equipped with magnets to facilitate apposition or thermal elements to allow for tissue fusion technology to be applied.

In some particular embodiments, the tip of the first catheter comprises a first convex contact area and a second convex contact area, the first convex contact area having when viewed in cross longitudinal section a curvature that is different from the one of the second convex contact area. The bearer of the second catheter comprises a first concave surface contact area and a second concave surface contact area, the shape of these concave areas being in print and counter print with the two convex contact areas of the first catheter.

The present disclosure also relates to a kit comprising a device as presented above and a tool that is to be guided in the internal lumen of the first catheter, the tool being a sharpened wire, or a RF wire that can allow the perforation of the two adjacent vessel walls and the navigation of the wire from the one vessel to the other and with the desired angle created by the two connected catheters.

### BRIEF DESCRIPTION OF THE DRAWINGS

The objects, advantages and other features of the present invention will become more apparent from the following disclosure and claims.

The following non-restrictive description of preferred embodiments is given for the purpose of exemplification only with reference to the accompanying drawings in which:
FIG.1 is a schematic view of a system for creating a fistula between two vessels, the system being represented in place within the two vessels, the system comprising two catheters, five cross sections of one of the two catheters being represented, the system being in a first position wherein the two catheters are spaced from each other;
FIG.2 is a schematic view of the system represented in figure 1, the system being represented in place within the two vessels, the system being in a second position wherein the two catheters are in contact with each other, a hole being formed in the walls of the two vessels, a wire passing through the two catheters;
FIG.3 is a schematic view of a distal tip of one of the two catheters (venous) represented in figures 1 and 2;
FIG.4 is a schematic view similar to figure 3, showing a partial longitudinal cross section of the distal tip of the catheter;

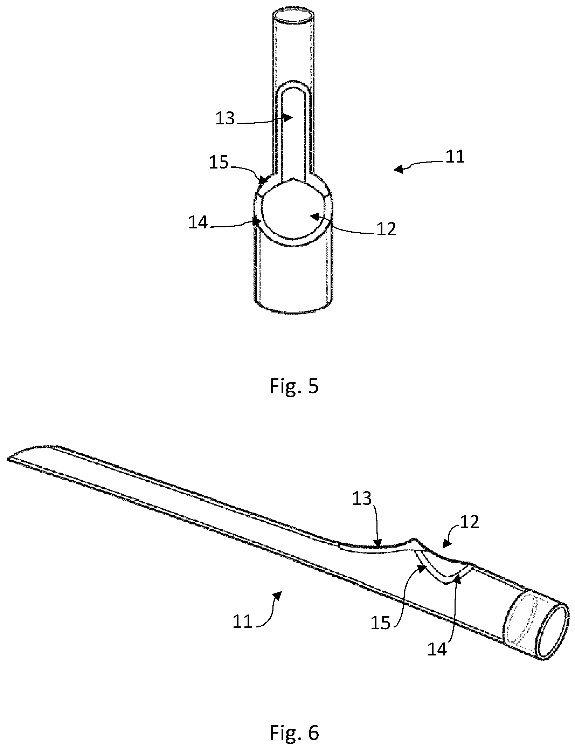

Figure 5 is a schematic view of a distal tip of one of the two catheters (arterial) represented in figures 1 and 2;
Figure 6 is a schematic perspective view of the distal tip of the catheter represented in figure 5.

### DETAILED DESCRIPTION OF ILLUSTRATIVE EMBODIMENTS

While methods and devices of the invention will be described with regards to forming vascular access sites for hemodialysis, the invention can be utilized to create other vascular access sites for other systems, for example, to create access sites in the respiratory system, digestive system and circulatory system.

The system represented in the figures is aimed to create a fistula between two vessels.

The system comprises two catheters, each being placed within one of the two vessels.

Placement of the catheter within the vessel can be done e.g. using the Seldinger technique, involving percutaneous puncture of a vessel with a hollow needle, introduction of a guidewire through the needle into the vessel lumen, removal of the needle while maintaining the guidewire in position, followed by advancement of the catheter over the guidewire. A sheath can be placed over the puncture site.

Guidewire have advantageously a hydrophilic coating to enhance their maneuverability.

The system enables the formation of percutaneous vascular anastomosis, in particular arteriovenous fistula (pAVF, endoAVF) for hemodialysis. AVF is advantageously created in the non-dominant arm, in the most distal vessels. The radiocephalic AVF is preferred, followed by the brachicephalic and the transposed brachial basilic vein fistula. Proximal radial artery AVF and ulnobasilic fistula can also be made. Proximal radial artery AVF is created by joining the median antecubital vein or cephalic vein with the radial artery. The system advantageously enable the formation of side-to-side anastomosis.

Vessel mapping is made using Doppler ultrasound or venogram. The inner diameter of the artery and vein should advantageously be more than 2.0 and 2.5 mm respectively.

In the following description the term "distal" is used to designate the extreme part of the catheters that is to be in the desired place for the fistula between the two vessels.

Each of the two catheters have a proximal part, not represented, that is to be placed outside the patient body.

As an example, the distal part of one catheter is placed within the proximal radial artery, whereas the distal part of the second catheter is placed within the deep communicating vein.

In the represented embodiments, the two catheters have a circular cross section, except for the extreme portion of their proximal end.

In the extreme portion of their proximal end, the two catheters have corresponding surface contact area.

In the represented embodiments, one catheter has a male extreme part, and the other catheter has a female extreme part, the shape of the two extreme parts being in print and counter-print.

The terms "male", "female" are used here by reference of the general shape of the two extreme parts of the catheters. The male extreme part can comprise groove, notch, slots corresponding to reliefs, lug, or pin on the female extreme part of the other catheter. The female extreme part can comprise reliefs, lug or pin corresponding to groove, notch, slots on the male extreme part of the other catheter.

Advantageously, the male extreme part and the female extreme part have indexing mean, enabling angular indexation of the male catheter and precise relative positioning of the two catheters.

In the represented embodiments, one catheter has a male extreme part that extends at the longitudinal end of the catheter, whereas the other catheter has a female extreme part that extends laterally.

The two catheters are thus movable between a first situation, in which the male catheter is spaced from the female catheter, and a second situation, in which the male catheter is placed in lateral contact with the female catheter.

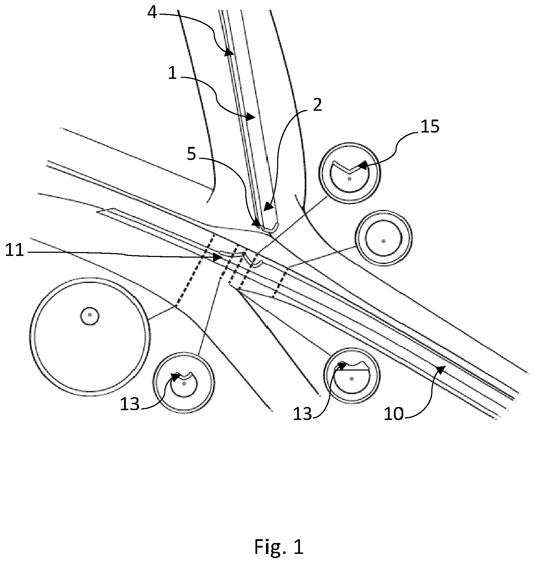

The first situation is represented schematically in figure 1, the distal end of the male catheter being placed in a vein such as the deep communicating vein, the distal end of the female catheter being placed in an artery such as the proximal radial artery. The distal end of the male catheter is spaced from the distal end of the female catheter.

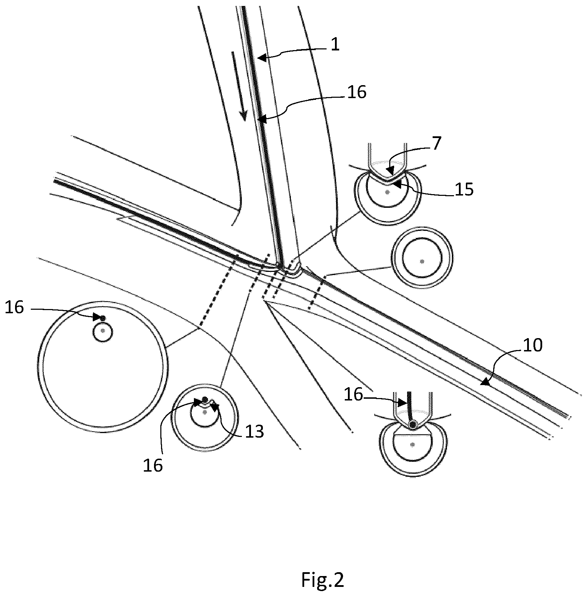

The second situation is represented schematically in figure 2, the distal end the male catheter being pressed against the distal end of the female catheter, the walls of the vein and artery being placed between the corresponding surface areas of the two catheters.

The male catheter has advantageously an internal lumen, with a distal exiting hole, for the passage of a wire. When the male catheter is in the situation represented in figure 2, action on the wire forms a hole in the walls of the vein and artery. Advantageously, the female catheter has a guiding surface for the wire exiting the male catheter.

In some embodiments, the hole is made by mechanical effort of the wire on the walls of the vein and artery.

In other embodiments, the internal lumen of the male catheter enables the passage of a cutting tool, a needle, a RF wire, or sharpened guidewire.

In variant or in combination, heat is applied on the walls of the vein and artery when the male catheter is pressed against the female catheter, for example using a radio frequency heat generator.

In the represented embodiment, the male catheter 1 has a distal end 2 having a cylindrical cross section, except for the extreme longitudinal portion that forms a convex tip 3.

The male catheter 1 has an internal longitudinal lumen 4 having an aperture 5 in the convex tip 3.

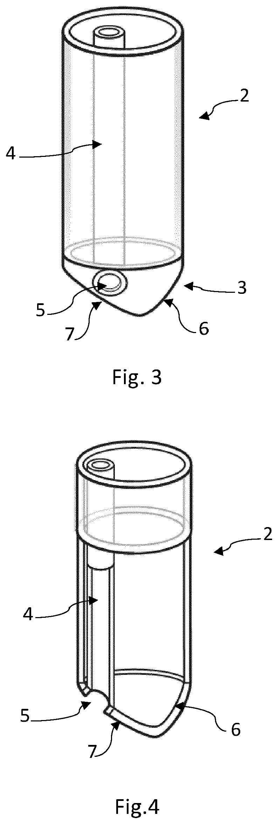

The convex tip 3 has a first surface contact area 6 and a second surface contact area 7. When viewed in cross longitudinal section, as in figure 4, the first surface contact area 6 has a curvature that is different from the one of the second surface contact area 7. In other words, the convex tip 3 does not form an external surface having a circular symmetry around an axis, in particular the longitudinal axis of the male catheter 1.

The aperture of the internal lumen lies within one of the two surface contact areas. In the represented embodiment, the aperture 5 lies within the second surface contact area 7.

The female catheter 10 has a distal end 11 comprising a lateral bearer 12 for the convex tip 3 of the male catheter 1.

The distal end 11 of the female catheter 10 has a lateral guiding surface 13 for a wire passing through the internal lumen 4 of the male catheter 1 and exiting at the opening 5.

The lateral bearer 12 comprise a first surface contact area 14 and a second surface contact area 15, the shape of these convex areas 14, 15 being in print and counter print with the surfaces 6, 7 of the convex tip 3.

Thus, when the convex tip 3 lies on the lateral bearer 12, the relative position of the two catheters 1, 10 is predetermined.

As represented on figure 2, a wire 16 guided by the internal lumen 4 and exiting at the opening 5 is deviated by the lateral guiding surface 13, after perforating the walls of the vessels.

Thanks to the shape of the convex tip 3 and the corresponding bearer 12, the opening 5 is turned in view of the extreme distal part of the female catheter 10.

The system enables endoAVF procedure, the anatomy associated with the AVF not been disturbed, and the vasa vasorum remaining intact.

Correct placement of the system within the vessels and correct puncture of the vessels are not dependent on the experience and skills of the surgeon. There is no need for radiopaque elements forming rotational indicators. The puncture of the artery is made in a direction that is fixed by the precise relative positioning of the two catheters.

The use of RF can be avoided, reducing the risks of intraoperative vessel spasm.

The procedure can be carried out by ultrasound guidance, angiographic imaging is not required. Magnets or tissue fusion can be used. Balloon angioplasty will include the outflow vein, the anastomosis and the pre-anastomotic artery which is not possible with the existing techniques. The anastomosis will be also created exactly at the level of the perforator which will lead to more flow to the superficial system.

Mean procedure time is short.

The endoAVF procedure is less invasive than surgery, and does not require general anesthesia. A period of several months may be required between surgical referral for access creation to a functional, surgically created AVF. This delay could influence some patients to start dialysis with a central venous catheter. Using the endoAVF procedure, this delay problem can be eliminated.

## Claims

1. Device for creating a fistula between two vessels, the device comprising two catheters (1, 10), each catheter having a proximal end and a distal end, the two catheters comprising corresponding surface contact areas in their distal ends, the surface contact area of a first catheter (1) extending in the tip (3) of the first catheter (1), the corresponding surface contact area of the second catheter (10) comprising a bearer (12) for the tip (3) of the first catheter (1), **characterized in that** the bearer (12) extends laterally in the distal end (11) of the second catheter(10), the two catheters (1, 10) comprising indexing means enabling precise relative positioning of the two catheters (1, 10) when the surface contact area of the first catheter (1) is against the surface contact area of the second catheter (10).

2. Device according to claim 1, **characterized in that** the first catheter (1) has an internal lumen (4) having an aperture (5) in said tip (3) for the passage of a tool, the second catheter (10) having a lateral guiding surface (13) facing said aperture (5) when the surface contact area of the first catheter (1) is against the surface contact area of the second catheter (10).

3. Device according to claim 2, **characterized in that** the surface contact area of the first catheter (1) is a male surface contact, the tip (3) of the first catheter (1) forming a convex surface area.

4. Device according to claim 3, **characterized in that** the tip (3) of the first catheter (1) comprises a first convex contact area (6) and a second convex contact area (7), the first convex contact area (6) having when viewed in cross longitudinal section a curvature that is different from the one of the second convex contact area (7).

5. Device according to claim 4, **characterized in that** the bearer (12) of the second catheter (10) comprises a first concave surface contact area (14) and a second concave surface contact area (15), the shape of these concave areas (14, 15) being in print and counter print with the two convex contact areas (6, 7) of the first catheter (1).

6. Kit comprising a device according to anyone of claims 1 to 5 and a tool that is to be guided in the internal lumen (4) of the first catheter (1), the tool (16) being a sharpened wire, or a RF wire.
